# EUROPEAN PATENT SPECIFICATION

(11) **EP 0 759 759 B1**
(45) Date of publication and mention of the grant of the patent: **12.11.2003**
(21) Application number: 95919786.4
(22) Date of filing: 11.05.1995
(51) Int. Cl.: C12N 15/11, C12Q 1/68, C07K 14/715, A61K 48/00, A61K 38/17, C12N 15/12

(54) **P75 TNF RECEPTOR PROMOTERS**
P75 TNF-REZEPTOR-PROMOTOREN
PROMOTEURS DU RECEPTEUR P75 DU FACTEUR DE NECROSE TUMORALE

(30) Priority: 11.05.1994 IL 10963394
(43) Date of publication of application: 05.03.1997
(73) Proprietor: YEDA RESEARCH & DEVELOPMENT COMPANY, LTD., 76100 Rehovot (IL)
(72) Inventor: WALLACH, David, 76406 Rehovot (IL); KUHNERT, Peter, Inst. of Veterinary Bacteriology, 3012 Berne (CH); EHRHARDT, Gotz, 78256 Steisslingen (DE); KEMPER, Oliver, 6719 Bockenheim (DE)
(74) Representative: VOSSIUS & PARTNER
(86) International application number: US9505853
(87) International publication number: WO95031206

(56) References cited:
- PETER KUHNERT ET AL.: "Cloning, sequencing and partial functional characterization of the 5' region of the human p75 tumor necrosis factor receptor-encoding gene (TNF-R)" GENE, vol. 150, no. 2, 15 December 1994, AMSTERDAM NL, pages 381-386, XP002033112
- PROC. NATL. ACAD. SCI. U.S.A., Volume 87, issued November 1990, KOHNO et al., "A Second Tumor Necrosis Factor Receptor Gene Product Can Shed a Naturally Occurring Tumor Necrosis Factor Inhibitor", pages 8331-8335.
- SCIENCE, Volume 248, issued 25 May 1990, SMITH et al., "A Receptor for Tumor Necrosis Factor Defines an Unusual Family of Cellular and Viral Functions", pages 1019-1023.
- MOLECULAR IMMUNOLOGY, Volume 30, Number 2, issued February 1993, ROTHE et al., "Genomic Organization and Promoter Function of the Murine Tumor Necrosis Factor Receptor Beta Gene", pages 165-175.
- GENE, Volume 134, Number 2, issued 08 December 1993, KEMPER et al., "Cloning and Partial Characterization of the Promoter for the Human p55 Tumor Necrosis Factor (TNF) Receptor", pages 209-216.
- SCIENCE, Volume 245, issued 28 July 1989, MITCHELL et al., "Transcriptional Regulation in Mammalian Cells by Sequence - Specific DNA Binding Proteins", pages 371-378.
- BIOTECHNIQUES, Volume 7, Number 3, issued 1989, SINGH et al., "Molecular Cloning of Sequence - Specific DNA Binding Proteins Using Recognition Site Probes", pages 252-261.
- PROC. NATL. ACAD. SCI. U.S.A., Volume 83, issued August 1986, KADONAGA et al., "Affinity Purification of Sequence-Specific DNA Binding Proteins", pages 5889-5893.
- SCIENCE, Volume 250, issued 16 November 1990, BIELINSKA et al., "Regulation of Gene Expression With Double-Stranded Phosphorothioate Oligonucleotides", pages 997-1000.
- 'Glossary' SCIENCE vol. 209, 1980, pages 1435 - 1438
- SHANNON M.F. ET AL: 'A novel tumor necrosis factor-responsive transcription factor which recognizes a regulatory element in hemopoietic growth factor genes' MOLECULAR AND CELLULAR BIOLOGY vol. 10, no. 6, June 1990, pages 2950 - 2959

## Description

### Field of the Invention

The present invention relates to a promoter sequence for the p75 tumor necrosis factor receptor (TNF-R), to its preparation and use.

### Background of the Invention

TNF is a multifunctional pro-inflammatory cytokine which affects a wide range of cellular functions. On the one hand, TNF is involved in the protection of the organism, but on the other hand, when over-produced, it can play a major pathogenic role in several diseases. TNF is known to be involved in inflammatory processes and to be a mediator of the damage to tissues in septic shock (1), graft-versus-host reactions (2) and in rheumatic diseases (3).

TNF exerts these effects by binding to two distinct cell surface receptors, which differ in size (about 55 and 75 kDa) and possess structurally dissimilar intracellular domains, suggesting that they signal differently (4-11). Almost all cells express TNF receptors (TNF-Rs), yet the amounts and relative proportions of the two receptors vary in different cell types. These variations are in part developmentally controlled; they are related to the phenotype of the cell and its state of differentiation, and in part can be induced transiently by cytokines and immune stimulatory components of pathogens (12-22). Studies of the function of the two TNF-Rs indicate that they have different yet interacting activities (23-28), and that their activity level may be correlated to the extent of their expression by the cell (29, 30).
A partial characterization of the promoter of the human p55 TNF receptor has been described (74).
These findings imply that mechanisms which affect the amounts and relative proportion of the TNF-Rs can have significant influence both on the nature and the extent of the cellular response to TNF and thus constitute important determinants of the physiological as well as pathological manifestations of the function of this cytokine.

In order to inhibit the deleterious effects of TNF, ways were sought which would interfere with the binding of TNF to its receptors. Thus neutralizing antibodies to TNF were raised (EP 186 833). Another approach to the inhibition of the action of TNF was the provision of soluble TNF receptors which compete with the cell surface TNF-Rs for the binding of TNF (EP 308 378 and EP 398 327).

Since binding to its receptors is required for TNF in order to exert its action, if less or no cell surface receptors are expressed, it should be possible to decrease or inhibit the deleterious effects of TNF. By the same token, it may be desired in certain cases to augment the beneficial action of TNF and in such a case this could possibly be achieved by increasing the amount of cell surface receptors expressed.

### Summary of the Invention

The present invention provides a promoter sequence of the human p75 TNF-R gene selected from a sequence which is located upstream of the 5' end of the gene and a sequence which is located in the first intron of the gene. The 5' upstream promoter sequence (called the 5' region promoter sequence) is contained within a 2.1 kbp sequence, and the intron promoter sequence is contained within a 1.9 kbp sequence. The 5' region promoter sequence is capable of controlling the expression of the native p75 TNF-R gene product, while the intron region promoter sequence is capable of controlling the expression of a truncated p75 TNF-R gene product. Further, the intron region promoter may also function as an enhancer element for the transcription of the p75 TNF-R gene. Moreover, the intron region promoter has upstream to the intron promoter a transcription inhibitory region, which may serve as a modulator for the expression of the truncated p75 TNF-R gene product.

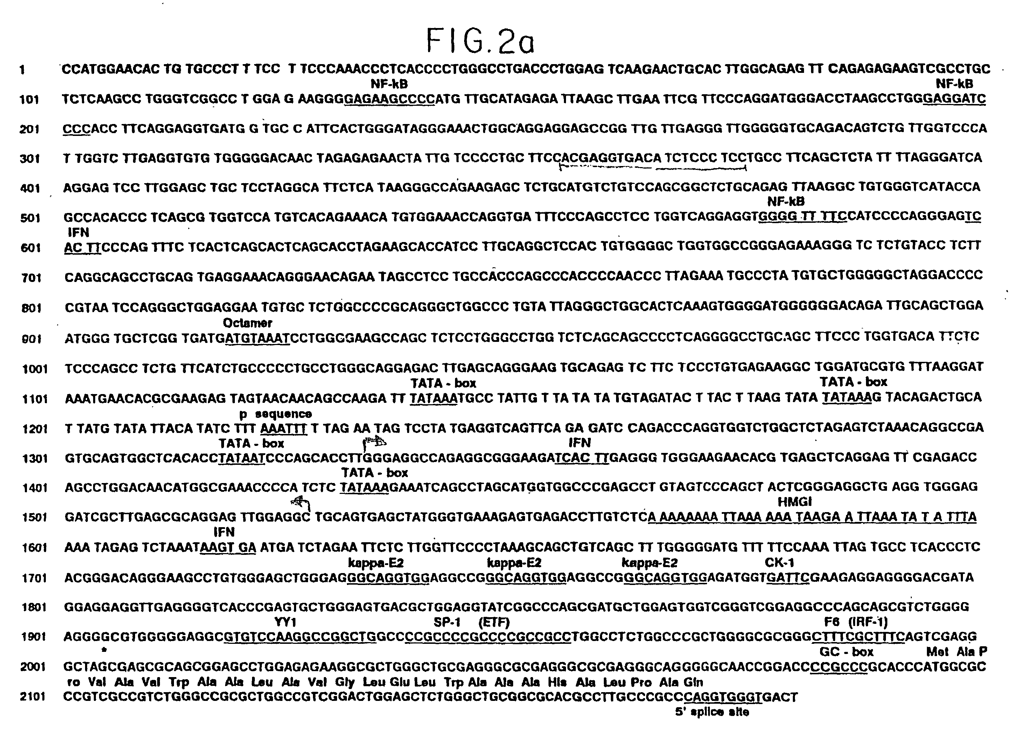

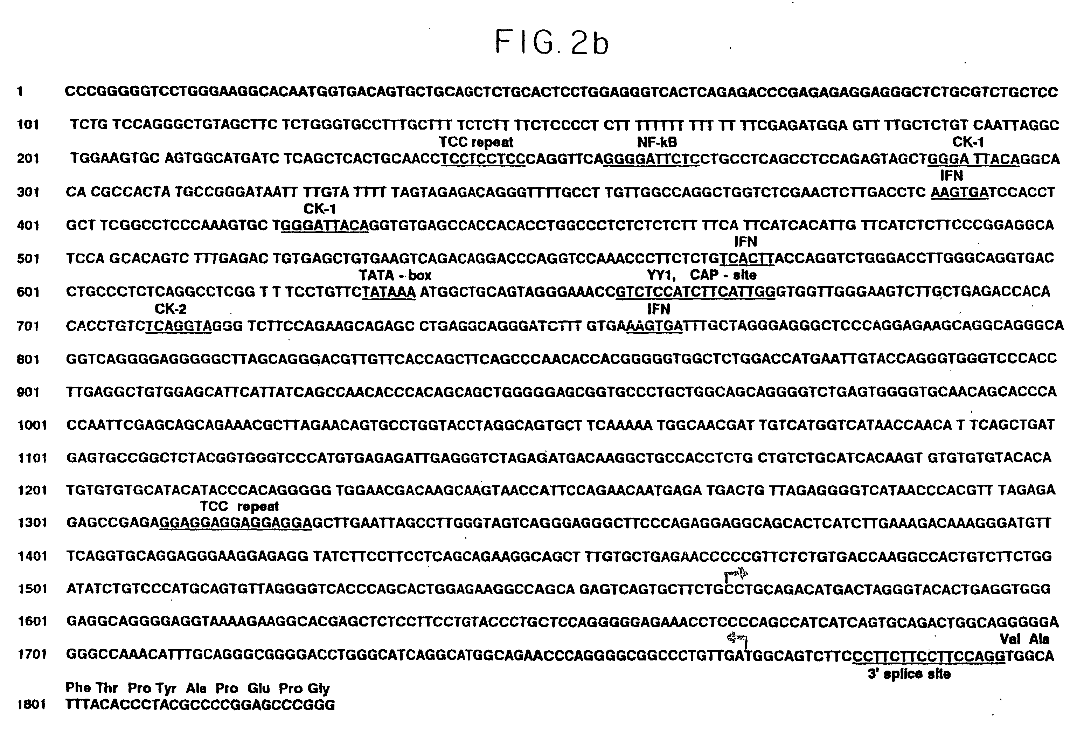

The invention in preferred embodiments provides the 2.18 kbp NcoI fragment, encoding the 5' region promoter sequence, and the 1.8 kbp SmaI fragment, encoding the intron promoter sequence, both fragments being derived from a genomic library containing the human p75 TNF-R. Other preferred embodiments provided by the invention are a portion of the 5' region promoter sequence containing the essential promoter activity and extending between at least nucleotides 1335 and 1527 of the sequence depicted in Fig. 2A; a portion of the intron promoter sequence containing the essential promoter activity and extending between at least nucleotides 1569 and 1768 or between at least nucleotides 1569 and 1827 of the sequence depicted in Fig. 2B; and a portion of the intron promoter region sequence containing the transcription inhibitory region and extending between nucleotides 1 and 1569 of the sequence depicted in Fig. 2B.

In another aspect, the invention provides sequence motifs present in the above described promoter sequences, including the promoters of the 5' upstream and intron regions as well as the transcription inhibitory region of the intron region. Such motifs have been shown to bind certain transcription factors which could be necessary for promoter activity and might be involved in regulation of this promoter.

The motifs may be prepared by deletion of the unwanted sequences upstream and/or downstream the desired motif in the full sequence, i.e. by employing restriction enzymes to cut the full promoter sequence to arrive at the desired motif, and the resulting motif can then be inserted into a vector together with suitable control sequences and other conventional means required in order to obtain a vector which, on insertion into a suitable prokaryotic strain is capable of expression of the desired motif on culturing of the strain.

The motif thus obtained can be used to screen, e.g. a human genomic library or a cDNA library for factors, e.g. transcription factors, binding thereto. Once these factors have been isolated, purified and identified by any conventional means, their inhibition should inhibit TNF-R formation, while their increased production should cause enhanced TNF-R expression thereby leading to the desired effects, i.e. inhibition or enhancement of TNF binding to its receptors.

Since the amount of specific transcription factors present *in vivo* is not unlimited, inhibition of TNF-R expression and, as a consequence, inhibition of deleterious TNF effects could also be achieved by the expression of a large number of motifs or motif regions. These will compete with promoters containing such motifs or motif regions for binding of the transcription factors. A "motif region" comprises the motif itself together with sequences flanking it on both sides, or several motifs connected by parts of the whole promoter sequence and flanked on both sides by sequence parts. It is to be understood that a motif or motif region according to the present invention, as noted above, and as set forth hereinbelow includes those present in both the active promoter regions and the transcription inhibitory region according to the invention. Likewise, the transcription factors of the invention include those which bind the active promoter regions and those which bind the transcription inhibitory region.

The present invention also provides pharmaceutical compositions comprising a sequence motif according to the invention.

In another aspect, the invention provides pharmaceutical compositions comprising a motif region according to the invention.

### Description of the Drawings

**Fig. 1** shows, schematically, the partial restriction map and various pBluescript subclones of the 5' region, including the 5' upstream region, the first exon, the first intron and the second exon, of the human p75 TNF-R gene, as described in Example 1.
**Fig. 2** (A and B) show, schematically, the nucleotide sequences of the 5' upstream promoter region (Fig. 2A) and the intron promoter region (Fig. 2B), as described in Example 2.
**Fig. 3** shows, graphically, the results of determination of the activity of the 5' upstream and intron promoter regions as determined in an assay in which these promoter regions were used to control the expression (given in relative light units - RLU) of the luciferase gene in expression vectors encoding luciferase, as described in Example 3.

### Detailed Description of the Invention

A human genomic library containing the human p75 TNF-R was obtained from Stratagene. This library was a phage library, from which 8 phages were positively identified as carrying the human p75 TNF-R gene, 6 of which phages were different. Various fragments from the positive phages were isolated, cloned and sequenced.

As is detailed herein below in the Examples, we have determined the sequence of the 5' region of the human p75 TNF receptor gene. We show that a 2 kb region 5' upstream of the published cDNA, as well as a region in the 3' part of the first intron contain promoter activity.
There are reports of different mRNA sizes for the p75-R and it would seem that this results not only from different 3' ends but also from different 5' ends. The finding of promoter activity in the regions of the gene that we have sequenced is strong evidence that we have cloned at least one of the promoters for the human p75 TNF receptor gene. Primer extension analysis and RACE will show where the 5' end of the gene is, and what products of the p75 TNF-R gene are obtained when expression of this gene is controlled by the different promoter regions. A number of clones have been derived (as set forth in Examples 1 and 2) which encode the whole 5' upstream promoter region or parts thereof or the whole intron promoter region or parts thereof. The activity of the 5' upstream and intron promoter regions was demonstrated by inserting these regions into a vector encoding luciferase, and subsequently showing that these were capable of positively controlling the expression of the luciferase gene (Example 3).

Examination of the promoter sequences located in the 5' upstream region and in the first intron region of the p75 TNF-R gene (see Example 2 below) reveals a variety of sequence motifs which may allow response to transcription factors which are affected by inducing agents, including, for example, TATA boxes, CAP sites and consensus sequences for AP-2 and NF-κB (see also Figs. 2A and 2B). These regulatory elements may allow for induced transient changes, superimposed on the pattern of constitutive expression of the receptor, perhaps by effects of certain cytokines which are formed at sites of inflammation.

Among the putative motifs discerned in the promoter regions (5' upstream and first intron regions), the following ones seem to be of particular interest: In the 5' upstream promoter region there are three canonical TATA boxes, two of which are followed very closely by CAP sites; three, closely located, and equaled spaced kappa-E2 motifs; four adjacent GC boxes; and binding motifs for several transcription factors which can mediate the effects of some inducing factors known to enhance p75 TNF-R expression, including NF-κB, the cytokine-2 motif and AP-2. In the first intron promoter region, particularly at the 3' end of the first intron there are, amongst others, a TATA box followed by a CAP site 25 bp. downstream and a threefold TCC repeat motif, which occurs twice in the p55 TNF-R promoter region.

Thus, it is assumed that the 5' upstream promoter region of the p75 TNF-R gene is the region necessary for the promoter activity of the p75 TNF-R gene, namely, to control the expression of the gene to ultimately result in the production of the normal p75 TNF-R receptor product, while the promoter region in the first intron is indicative that the p75 TNF-R gene encodes other forms of the receptor. Moreover, the promoter region in the first intron may also function as an enhancer element for the transcription of the p75 TNF-R gene. Furthermore, the promoter region in the first intron also contains a transcription inhibitory region upstream of the active intron promoter region, which inhibitory region may be involved in the modulation of expression of the other forms of the p75 TNF-R.

The invention is illustrated by the following non-limiting examples:

### Cell lines and culture conditions:

The baby hamster kidney (BHK) cells were grown and maintained in standard BHK cell-culture conditions (DMEM + 10% FCS + 2 mM glutamine).

### EXAMPLE 1

### Cloning and Analysis of clones containing the 5' region of the p75 TNF-R gene

Two human genomic libraries were screened (using standard hybridization methods) with the full-length cDNA encoding human p75 TNF-R (also known as TNF-RII). This cDNA contained the sequence from bases 90-1475 of the sequence according to Smith et al. (10). The following human genomic libraries in the form of recombinant phage libraries were employed: An ATCC library (ATCC No. 57738) specific for human chromosome 1; and a Stratagene human lymphocyte library (Stratagene No. 943202). Several screenings of the ATCC library did not reveal any positive phages, i.e. no phages containing a sequence hybridizable with the p75 TNF-R cDNA probe were contained in the library. Screening of the Stratagene library revealed 8 positive phages, 6 of which differed from each other as shown by restriction enzyme comparative analysis (results not shown).

Two of the above different positive phages, called U and H, were further examined by hybridization to different fragments of the p75 TNF-R cDNA: Both U and H hybridized to a 250 bp. fragment of the 5' end of the cDNA including the untranslated region (UTR), but did not hybridize to a somewhat shorter 160 bp. fragment cDNA, contained within the above 250 bp fragment at its extreme 5' end. A third phage, of the above different phages, called G, was isolated with the aforesaid 160 bp. fragment of the 5' region of the cDNA. Thus, G also encodes a 5' region of the p75 TNF-R gene.

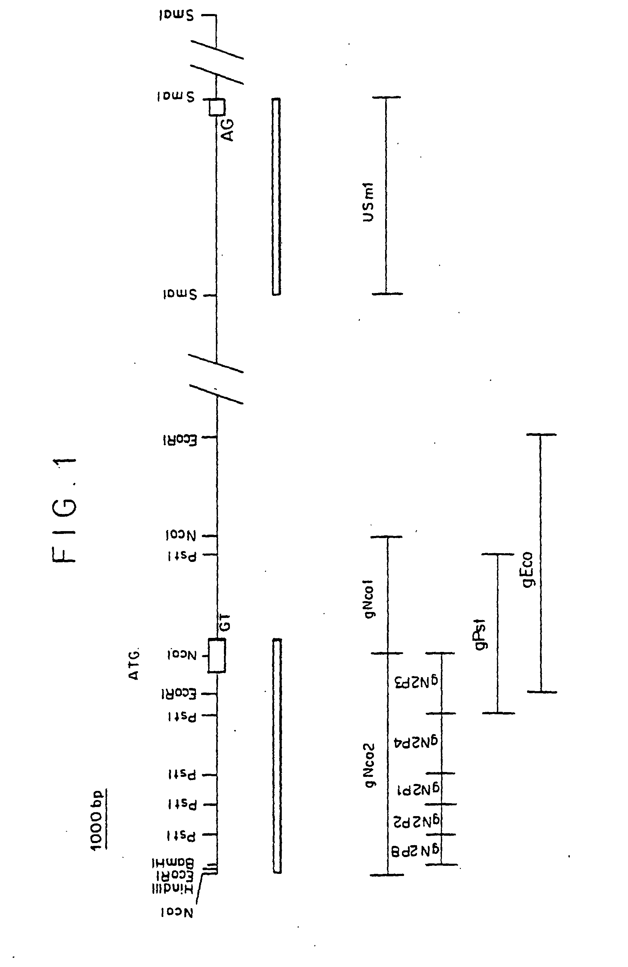

Further characterization of the 3 phages (U, H, G) by hybridization to the 5' cDNA fragments (probes) revealed that: (i) with U and H, two SmaI restriction fragments, obtained from the p75 TNF-R sequence contained in these phages of sizes 1.9 and 3.6 kb, hybridized to the above noted 250 bp. cDNA probe; and (ii) with G, the above noted 160 bp. cDNA probe hybridized to two NcoI fragments, of sizes 2 and 1.1. kb, and also hybridized to a 2.4 kb EcoRI and a 1.5 kb PstI fragment, all of these NcoI, EcoRI and PstI fragments being obtained from the p75 TNF-R sequence contained in the phage G. All of the above fragments from phages U, H and G which hybridized to the 250 or 160 bp. cDNA probes were then subcloned into plasmids. Subcloning was by way of standard procedures using the plasmid pBluescript (Stratagene). Following subcloning, the various subcloned fragments of phages U, H and G were analyzed by restriction enzyme analysis to obtain a restriction map. In Fig. 1 there is shown, schematically, the restriction map (partial) of the plasmid clones which cover the 5' region of the human p75 TNF-R gene. It should be noted that the plasmids shown in Fig. 1 were derived from phage G (gNco1, gNco2, gPst, gEco, and the various subclones of gNco2 shown under gNco2), while the SmaI clone (USm1) was derived from phage U. In Fig. 1 the black bars indicate the regions that have been presently sequenced (i.e. from subclones gNco2, gNco1 and USm1-see Example 2 below); the filled boxes indicate the regions of the known cDNA sequence of Smith et al. (10); and the exon/intron boundaries are indicated by "GT" (start of intron I / end of exon I) and "AG" (start of exon II / end of intron I).

In order to assure that the phages, of the above Stratagene library, represent the genomic organization of the p75 gene and are not the result of recombination, the following tests (or "check-ups") were performed:
(i) That the 1.9 kb SmaI fragment represents the genomic SmaI fragment can be shown indirectly by the fact that two independent phages (U and H) contain the same fragment which hybridizes to the 250 bp. cDNA probe.
(ii) To test whether the single phage isolate, G, of the very 5' region (i.e. extreme 5' end of the p75 TNF-R gene) represents the genomic organization, a comparison of the phage restriction pattern with the one of the native genomic DNA from U937 cells was made by digestion of the phage and genomic DNA with BamHI, EcoRI and PstI, followed by hybridizations with the 2 kb. NcoI fragment (from subclone gNco2). Comparison of the genomic and phage restriction patterns and hybridization of the restri ion fragments to the 2 kb. NcoI fragment probe showed that both the phage and the genomic DNA had the same restriction pattern with the same fragments hybridizing to the probe (results not shown). Thus the single phage isolate, G, represents the actual genomic organization of the 5' region of the p75 TNF-R gene.

Moreover, from the above cloning, hybridization and restriction analysis, the following facts are revealed: a) by using the first part (90-1475 bp) of the published cDNA as a probe, 8 independent phages (6 of which differed from each other) can be isolated from the Stratagene ibrary, each having an insert of about 15-20 kbp., this being indicative that the p75 TNF-R gene is rather large and is spread over several dozen kbp; and b) in view of the fact that phages U and H, while independent, did not contain the very 5' region of the p75 TNF-R gene, the first intron (intron I) is also rather long (> 10 kb).

### EXAMPLE 2

### Sequence and characterization of the 5' region of the p75 TNF-R gene

To elucidate the structure of the 5' region of the p75 TNF-R gene, i.e. the location and nature of the promoter region, the various clones (subclones in pBluescript - see Example 1, above) were either partially or completely sequenced (see black bars in Fig. 1). In particular, clones gNco2 and USm1 were completely sequenced, while clones gNco1, gPst and gEco were partially sequenced. In Fig. 2 (A and B) there are shown, schematically, the sequences of the two parts of the 5' region of the p75 TNF-R gene that have been fully sequenced. Sequencing was by standard procedures in which the above clones in pBluescript were sequenced from both directions either with the Sequenase Version 2.0 kit (USB), or automatically with the Applied Biosystems Automated Sequencer (Model 737A DNA Sequencer, Applied Biosystems, USA). Following the sequencing, general sequence analysis was performed using the GCG (Genetics Computer Group, Madison, U.S.A., standard computer software package) package and specific sequence analysis, i.e. identification of transcription factor binding motifs, was carried out with the FINDPATTERNS option of the above standard software package. In Fig. 2A there is depicted the sequence of the very 5' region of the p75 TNF-R gene, including the first exon and exon/intron boundary (the first exon sequences and exon/intron boundary being determined by taking into consideration the cDNA sequence of Smith et al. (10)). The amino acid sequence of the cDNA is indicated above the nucleotide sequence. Underlined are four canonical TATA-boxes as well as other transcription factor binding sites (e.g. kappa-E2 sites and GC-boxes). The asterisk (*) indicates the start of the cDNA according to Smith et al. (10). In Fig. 2B there is depicted the sequence of the end of the first intron and part of the second exon (this also based on information from the Smith et al (10) cDNA sequence). Underlined are putative TATA-box, S1 nuclease hypersensitivity site (S1-HS), which is a TCC repeat, CAP-site, and various other putative transcription factor binding sites. The amino acid sequence of the cDNA is indicated above the nucleotide sequence (i.e. this is the start of the second exon).

The sequence analysis showed the following characteristic features of the 5' region of the p75-TNF-R gene:
(i) At the extreme 5' end of the p75-TNF-R gene (i.e. sequence of Fig. 2A, and indicated sequenced region of various subclones of phage G shown in Fig. 1 and described in Example 1): In this region a comparison of the cDNA sequence with our genomic 5' region clone showed that bases 1-166 of the cDNA sequence are identical to our sequence at same region, the 3' end of this region containing the coding sequence for amino acids 1-26, and continuously followed by the intron sequence with the characteristic GT dinucleotide consensus splice site at the 5' border of the intron. The aforesaid sequence is preceded by the 89 nucleotide long 5' untranslated region (UTR) of the published cDNA sequence (10). Since a similar UTR length has been described by another group for the p75-R cDNA (35) it is likely to correspond to the genuine size of the p75 TNF-R message. The first nucleotide in this UTR sequence, which may be the site of transcription initiation, is indicated with an asterisk. The sequence upstream of it contains three canonical TATA-boxes (at positions 1139, 1183 and 1431 of the sequence of Fig. 2A) and in addition, one unconventional TATA-box at position 1318. Two of the TATA-boxes, at positions 1318 and 1431, are followed by CAP-sites.
   However, in both cases, the distance between the CAP-site and the TATA-box is unusually small (5 bp). As to which one (or all) of the above TATA boxes is functionally active can be determined by standard analysis. No CCAAT-motif is present in the sequence. A sequence similar to the initiator-motif, shown to be involved in definition of the transcription start site (36), is located at position 1918 (YY1). Two, overlapping GC-boxes are present at positions 1938 and 1943 and two others, at positions 2082 and 2164 of the sequence of Fig. 2A. The two overlapping GC-boxes reside within a 22 bp. GC-stretch. Such long GC sequences have been proposed as binding sites for the transcription factor ETF (37, 38). At position 1566, there is a 31 bp. AT-rich sequence which can serve as an HMGI-binding motif. In certain promoters, such motifs have been demonstrated to contribute to promoter efficacy (39). A 3-fold repeat of the kappa E2 motif, which constitutes part of the enhancer of the immunoglobulin kappa light chain gene (40) is found at positions 1732, 1747 and 1762 of the sequence of Fig. 2A. The three repeats are very closely and equidistantly spaced by the same two hexanucleotides (AGGCCG).
   Among the putative transcriptional factor binding motifs in the 5' flanking sequence, there are several which could confer response to agents which were shown to affect the expression of the p75-R. These include an NF-κB motif (32) at positions 130 (reverse), 194 (reverse) and 577 and a site homologous to the cytokine-1 motif (41) overlapping to the NK-κB motif at position 194, of the sequence of Fig. 2A. NF-κB sites can confer responsiveness to TNF and IL-1 (42), while the cytokine-1 motif binds TNF-induced factors. Both TNF and IL-1 have been shown to enhance the expression of the p75-R in certain cells (43, 44). A hexanucleotide which, in a repetitive form constitutes an interferon-responsive motif, AAGTGA (45) resides at positions 599 (reverse), 1356 (reverse) and 1616. A recently defined consensus sequence, the F6 consensus G A/G A A G C N G A A A G which binds an interferon-induced transcription factor, IRF-1 (46) is located at position 1982 (reverse). The consensus site for AP-2, T/C C G/C C C A/C N G/C G/C G/C (47) is found at position 1938, overlapping with the potential ETF site, and reverse copies of this element are located at positions 269, 874 and 1904 of the sequence of Fig. 2A. However, since this consensus sequence consists almost entirely of degenerate bases, its significance is uncertain. AP-2 is involved in the response to PMA as well as to cAMP (33). Both agents have been shown to induce the p75 TNF-R messenger (48, 49, 50). A sequence similar to the CAMP responsive element, TGAGTCA (51) occurs at position 1242, albeit with two mismatches. The p75 messenger was also reported to be induced upon T and B cell activation.
   In this process, Octamer binding factors (for B and T cells) (52, 53) and a factor binding the P sequence of the IL-4 promoter, CGAAAATTTCC, (for T cells) (54) have been shown to play a role. A sequence similar to the octamer-motif occurs at position 918, ATGTAAAT and the P core sequence, AAATTTTT, at position 1222, of the sequence of Fig. 2A.
(ii) The intron sequence: This first intron has only been partially sequenced (see black bars in Fig, 1, the end of the sequence in Fig. 2A - i.e. starting from the GT dinucleotide, and then, separately, after a long (about 10 kb) unsequenced stretch, the entire sequence in Fig. 2B up to and including the AG dinucleotide, immediately before the indicated translated sequence).

Comparison of the cDNA with the sequence of the cloned 1,828 bp. genomic SmaI fragment (Fig. 2B) revealed that it contains at its very 3' end the continuation of the cDNA, again with the characteristic AG dinucleotide at the intron's 3' border, i.e. a 3' consensus splice site:
(C/U)₁₁NCAG (55), which precedes the coding sequence at position 1795 of the sequence of Fig. 2B, starting with Val27 in the leader of the p75 TNF-R. Therefore it is assumed that this sequence is part of the first intron of the p75 TNF-R gene. Surprisingly, within this intronic sequence (see underlined sites in Fig. 2B), we could define several sites or regions which are characteristic of promoters and cytokine inducibility: At position 630, there is a canonical TATA-box, which is followed, 26 bp. downstream, by a sequence similar to the initiator-binding site YY1, (36). Although no CCAAT-motif is discernible upstream of the TATA-box, an inverted copy of the element occurs at position 668 just downstream of the initiator motif. A TCC-repeat motif, found in various proto-oncogene and housekeeping-promoters, as well as twice in the p55 TNF-R promoter(56), occurs at positions 238 and 1310 (inverted), of the sequence of Fig. 2B.

Also, the intronic sequence contains consensus sites for transcription factors which may take part in regulation of p75-R expression. These include an NF-B consensus sequence (32) at position 255, two copies of a form of the cytokine-1 motif (which was also reported to respond to TNF) as it occurs in the human GM-CSF promoter (41), at positions 288 and 422, an interferon-responsive sequence, AAGTGA (45) (see above), at positions 388, 567 (reverse) and 757, a cytokine-2 element at position 709 (57), and three inverted copies of the AP-2 consensus sequence G G G / C C A / T G / C G /C C (47) at positions 359, 406 and 774. A consensus site for glucocorticoid receptor, AGAWCAGW (58) can be discerned at position 1025. This site may contribute to the reported upregulation of p75-R mRNA in the U937 cells by glucosteroids (59). A sequence which resembles the cAMP-responsive element TGACGTCA (51), but for two mismatches, can be found at positions 381 and 530.

The unexpected finding of promoter activity in an intron of the p75 TNF-R gene points to possible existence of additional translation products of this gene, besides the known form of the receptor. There are a number of ATGs downstream to the putative initiation binding motif in this sequence. The one occurring before the longest ORF (150 bp.) at position 874, is located within an almost perfect Kozak-box. The deduced 50 amino acid sequence starting at this point showed no similarity to any of the proteins found in Swiss-Protein data base (checked using the FASTA program [GCG, 1991 - see ref. no. 60). At position 1768, close to the 3' end of the intron, there is an ATG start codon, in-frame with the receptor's coding sequence. The sequence surrounding this ATG does not fit very well to the consensus sequence proposed by Kozak (61) since it has a T in position -3, which occurs only in 1% of the 699 sequences analyzed by Kozak. However, also the translation start of the p75 TNF-R does not match precisely the consensus sequence, the C in its -3 position occurring only in 3% of the sequences. Translation starting at this "intronic" ATG would give rise to a truncated form of the receptor, in which the 26 N-terminal amino acids, which constitute a major part of the leader sequence of the described TNF-R, are replaced by 9 other residues, which may result in this protein product being an intracellular protein. It should also be noted that a protein known to be secreted may also exist in an intracellular form as was shown for the IL-1 receptor antagonist. Yet another possibility could be that translation of the mRNA starts at a Met within the cDNA part downstream of the SmaI clone, thus encoding a receptor which lacks, not only the leader sequence, but also part of the extracellular domain.

In any event, from the above analysis it seems likely that the promoter region within the intron sequence of the p75 TNF-R gene encodes two new protein products; a new protein and a new leader. However, our recent deletion analysis of the cloned first intron region, in particular the cloned approx. 1.9 kb intron region (see below, Example 3) revealed that upstream to the proposed new protein, there is no active promoter, and hence it is not apparent how such a new protein may be expressed. Accordingly, such a new protein encoded by a sequence (the observed ORF) within the first intron of the p75-TNF-R gene, is probably not expressed. The new leader sequence does however appear to be highly feasible in light of this recent deletion analysis, which shows that a strong promoter is present just upstream to the start of the leader.

The following is the sequence of this new leader, the nucleotide numbering corresponding to that shown in Fig. 2B.

NEW LEADER
1768/1
ATG GCA GTC TTC CCT TCT TCC TTC CAG GTG GCA TTT ACA CCC TAC
Met ala val phe pro ser ser phe gln val ala phe thr pro tyr
GCC CCG GAG CCC GGG
ala pro glu pro gly

It should be noted that in the above sequence the sequence numbering starts with the nucleotide sequence number as in Fig. 2B and is followed by the amino acid sequence number, e.g. in first sequence (new leader) the nucleotide sequence is from 1768 to 1827 and amino acid sequence is 1-20.

Furthermore, it is also possible that the intron region promoter sequence, besides having the capability for controlling the expression of the above noted new leader product, may also function as an enhancer element for the transcription of the p75 TNF-R gene. Such downstream enhancers are well known in many eukaryotic genes.

Moreover, it has now been found (see Example 3 below) that the intron promoter region contains a transcription inhibitory region upstream of the active intron promoter, this inhibitory region possibly being involved in the modulation of expression of the p75 TNF-R gene, in particular, the expression of a new p75 TNF-R product which starts from the above new leader, that is a truncated form of the native p75 TNF-R.

To further clarify the above sequence analysis of the two promoter regions of the p75 TNF-R gene (the 5' upstream and the intron promoters), the following is a summary of the comparison of the sequences from clones G (5' upstream promoter region) and U (intron promoter region) and known sequence motifs, being indicated as "ref."

The motifs are presented in two groups: those found in the exact form reported by others ("no mismatch") and those that differ slightly (mismatch"). Thus for each motif, the following information is given: The name of the motif and its reported nucleotide sequence; and the related sequences which were found in the p75-R promoter sequences in accordance with the present invention.

The above information is presented in the following way:
(i) The phage in which we observed the related motif. G designates the phage clone in which 5' upstream promoter sequence was found. U designates the phage clone in which the promoter sequence within the intron was found;
(ii) The positions within the sequence with reference to Figs. 2A (clone G) and 2B (clone U) in which we observed the motif and the exact sequence of the motif; and
iii) ref: reference to the publication in which the motif was defined (including the exact sequence thereof).

It should be noted that some of the motifs listed below are not described herein above and represent additional putative motifs of the 5' upstream and first intron promoters:

no mismatch

insulin
insulin
GTGGAAA
G 537 : AACAT GTGGAAA CCAGG
ref :
insulin 0 GTGGAAA Cell 45 : 35-44 (1986)

AP-2
G :
AP-2_CS4 YCSCCMNSSS
1,938 : TGGCC CCGCCCCGCC CCGCC
1,943 : CCGCC CCGCCCCGCC GCCTG

AP-2_CS4 / Rev SSSNKGGSGR
269 : GTTGA GGGTTGGGGG TGCAG
874 : AAGTG GGGATGGGGG GACAG
1,904 : GGAGG GGCGTGGGGG AGGCG
1,905 : GAGGG GCGTGGGGGA GGCGT

AP-2_CS5 GSSWGSCC
840 : CGCAG GGCTGGCC CTGTA
1,335 : ACCTT GGGAGGCC AGAGG
1,930 : AGGCC GGCTGGCC CCGCC
1,953 : CCGCC GCCTGGCC TCTGG
AP-2_CS5 / Rev GGSCWSSC
839 : CCGCA GGGCTGGC CCTGT
856 : TATTA GGGCTGGC ACTCA
U :
AP-2_CS4 / Rev SSSNKGGSGR
1,691 : AGACT GGCAGGGGGA GGGCC

AP-2_CS5 / Rev GGSCWSSC

359 : TTGTT GGCCAGGC TGGTC
406 : GCTTC GGCCTCCC AAAGT
774 : AGGGA GGGCTCCC AGGAG
ref :
AP-2_CS4 0 YCSCCMNSSS (33)
AP-2_CS5 0 GSSWGSCC (47)

AP-3
G:
AP-3_CS TGTGGWWW
536 : AAACA TGTGGAAA CCAGG
ref :
AP-3_CS 0 TGTGGWWW Nucleic Acids Res. 20 : 3-26 (1992)

NF-kB
G :
NFkB_CS1 GGGRHTYYHC
577 : GAGGT GGGGTTTTCC ATCCC
NFkB_CS1 /Rev GDRRADYCCC
130 : AAGGG GAGAAGCCCC ATGTT
194 : CCTGG GAGGATCCCC ACCTT
NFkB_CS4 GGGRNTYYC
577 : GAGGT GGGGTTTTC CATCC

U :
NFkB_CS1 GGGRHTYYHC
255 : GTTCA GGGGATTCTC CTGCC
256 : TTCAG GGGATTCTCC TGCCT

NFkB_CS4 GGGRNTYYC
256 : TTCAG GGGATTCTC CTGCC

NFkB_CS4 /Rev GRRANYCCC
1,460 : GTGCT GAGAACCCC CGTTC
ref :
NFkB_CS1 0 GGGRHTYYHC (32)

NFkB_CS4  0  GGGRNTYYC  Hematol Bluttransfus 32:411-415
(1989)

CK-2 (cytokine-2)
U :
CK-2                    TCAGGTA
          709 :  CTGTC  TCAGGTA  GGGTC
ref :
CK-2      0 TCAGGTA  (57)

INF
G :
INF.1                   AAGTGA
        1,616 :  TAAAT  AAGTGA  ATGAT
INF.1 /Rev                                TCACTT
          599 :  GGGAG  TCACTT  CCCAG
        1,356 :  GAAGA  TCACTT  GAGGG

U :
INF.1                   AAGTGA
          388 :  ACCTC  AAGTGA  TCCAC
          757 :  TGTGA  AAGTGA  TTTGC

INF.1 /Rev              TCACTT
          567 :  CTCTG  TCACTT  ACCAG
ref :
INF.1           0 AAGTGA  (45)
SP-1
G :
Sp1-ras1.3 /Rev         GCCCCGCCCC
        1,935 :  GGCTG  GCCCCGCCCC GCCCC
        1,940 :  GCCCC  GCCCCGCCCC  GCCGC
ref :
Sp1-ras1.3      0  GGGGCGGGGC  Science 232 : 1410-3 (1986)


IL-6
G :

F6_CS /Rev                STTTCNTTYC
          18 :  GTGCC  CTTTCCTTCC  CAAAC

IL-6_RE1 /Rev               TTCCCAG
          169 :  ATTCG  TTCCCAG  GATGG
          553 :  GTGAT  TTCCCAG  CCTCC
          603 :  GTCAC  TTCCCAG  TTTCT
U :
IL-6_RE1                    CTGGGAA
           11 :  GGGTC  CTGGGAA  GGCAC

IL-6_RE1 /Rev               TTCCCAG
        1,357 :  AGGGC  TTCCCAG  AGGAG

;IL-6_RE1 0  CTGGGAA                MCB13, 1183 (93/FEB)
ref :
F6_CS            0  GRAANGAAAS    (46)
;IL-6_RE1 0  CTGGGAA                 MCB13, 1183 (93/FEB)

oct2 (B cell development)
OCT 2                       ATGYAAAW
          918 :  TCATG  ATGTAAAT  CCTGG
ref :
OCT 2            0  ATGYAAAW      (52)

GCRE (glucocorticoid receptor)
GCRE /Rev                  GAGTCA
          56 :  CCCTG  GAGTCA  AGAAC
         596 :  CCAGG  GAGTCA  CTTCC

U :
GCRE /Rev                  GAGTCA
       1,553 :  CAGCA  GAGTCA  GTGCT
ref :
GCRE                   0  TGACTC       Cell 43 : 177-88 (1985)

mismatch

cAMP
1                    TGACGTCA
      1,242 :  TCCTA  tgaggtca  GTTCA mis=1
U :
1                    TGACGTCA
        381 :  ACTCT  tgacctca  AGTGA  mis=1
        530 :  AGCTG  tgaagtca  GACAG  mis=1
ref :
      cAMP_RE    0  TGACGTCA      (51)
NF-P (T cell development)
T-ACT /Rev               AAATTTTcc
      1,222 :  TCTTT  AAATTTTTAGAA

ref :
T-ACT                0   AAAATTTCC      (54)

Vitamin D (VDRE)
gnco2.seq x vdre        April 10, 1994    17:54.....
        1536   GCTATGGGTGAAAGAG   1551
           1   GCACTGGGTGAATGAG   16

ref :
VDRE          0   GCACTGGGTGAATGAGGACATTACTGAC     J Cell
                                    Biochem  49 :37

P53
p53_RS                    AGGCATGCCT
G         1,708 :  GGGAC  agggaagcct  GTGGG  mis=2
p53_RS                    AGGCATGCCT
U         1,573 :  CCTGC  agacatgact  AGGGT  mis=2
U         1,736 :  GCATC  aggcatggca  GAACC  mis=2
ref :
p53_RS    0  AGGCATGCCT       Nature Genet 1 : 45-9 (1992)

YY1 (initiator)
G :                       gtctccannnnnnnng
YY1       1,918 :  GAGGC  gtgtccaaggccggctg  GCCCC  mis=1

U :                    gtctccannnnnnnnng
YY1        656 :  AAACC GTCTCCATCTTCATTGG  GTGGT
ref :
 YY1        0  gtctccannnnnnnnng       (36)

CK-1
G :
1 /Rev                  GTGGAATCTC
        360 :  ACGAG  gtgacatctc  CCTCC  mis=2

U :
1 /Rev                  GTGGAATCTC
        255 :  GTTCA  ggggattctc  CTGCC  mis=2
ref :
CK-1           0  GAGATTCCAC       (57)

### EXAMPLE 3:

### Functional analysis - Promoter activity of the 5' region and the first intron region of the p75 TNF-R gene

In order to gain first insight into the functional significance of some 5' regions of the p75 receptor gene, several regions were cloned into a vector carrying the luciferase reporter gene. The constructs were transfected into BHK cells, which in our hands proved to be the most convenient system for having a first idea about promoter activity. Even though our main interest was in the very 5' part of the p75-TNF-R gene, the finding of a putative TATA-box and CAP-site in the intron prompted us to test also this region in a functional assay.

For the functional assay of the promoter(s) of the p75 TNF-R gene the following procedure was performed: BHK cells were transfected by the calcium-phosphate method according to Sambrook et al. (31). In each experiment, 10 g of plasmid DNA was transfected to semi-confluent 6 cm dishes. After overnight (8-12 hrs) incubation, the cells were washed 3 times with PBS and supplemented with fresh medium. After further 24 hours incubation, cells were harvested and luciferase activity in lysate supernatants was determined, using a Lumitron Luminometer set on 10 sec integration (62). Values were calculated as relative light units (RLU). The various vectors encoding the luciferase gene had cloned into them different promoters to control expression of the luciferase gene as follows:
i) CMV: positive control (luciferase gene under control of the CMV-promoter); LUC-0: negative control (luciferase gene without regulating sequence); gBX: 5' region of p75-TNF-R gene (nt 1-2089 in Fig. 2A) without ATG in "sense" orientation; gBS: 5' region (nucleotide 1-2089 in Fig. 2A) without ATG in "antisense" orientation; UsBX: intron fragment (nt 1-1827 in Fig. 2B) in "sense" orientation; UsBH: intron fragment (nt 1-1827 in Fig. 2B) in "antisense" orientation; UBX: intron fragment (nt 1-869 in Fig. 2B) without ATG in "sense" orientation; UBS: intron fragment (nt 1-869 in Fig. 2B) without ATG in "antisense" orientation.
   It should be noted that the various putative promoter-carrying fragments (and control promoters and fragments), as noted above, were cloned into the promoter-less luciferase vector LUC0, which contains the luciferase gene (63) replacing the CAT gene in the expression vector pBLCAT6 (64).

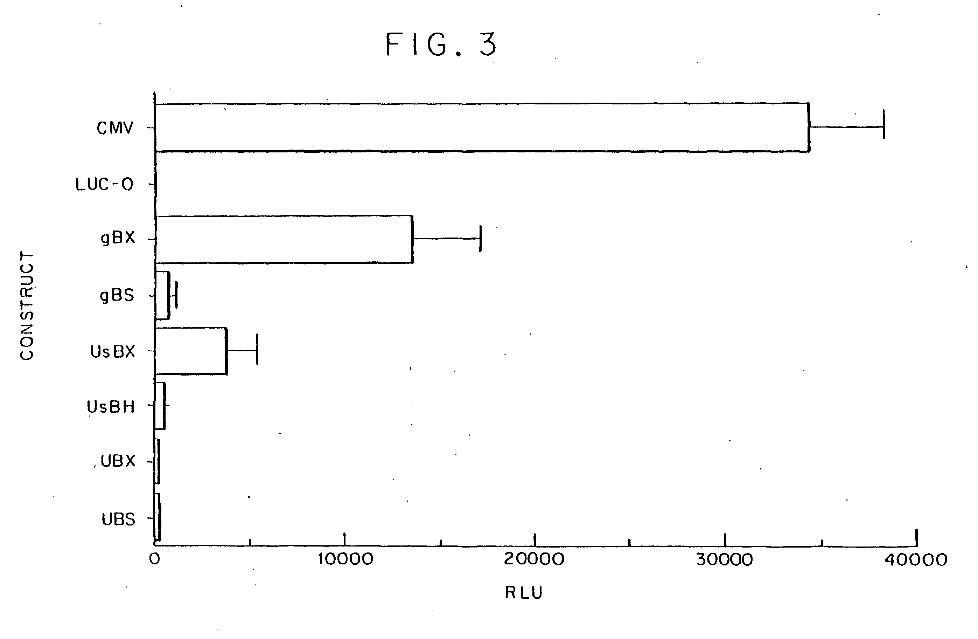

   Figure 3 shows the average results (standard deviation) of 3 independent transfections and subsequent luciferase assays. The very 5' region (gBX) shows very high activity compared with the positive control (CMV promoter). The promoter activity of this region is orientation dependent, as can be shown with the same fragment in antisense orientation (gBS) where a sharp drop in activity is observed. Interestingly, the intron region (UsBX) also shows significant promoter activity, here too the internal control in antisense orientation (UsBH) shows much less activity. This activity of the intron promoter is no longer detectable when only the part upstream of the first ATG (UBX or UBS) is linked to the luciferase gene, indicating that the observed promoter activity is mainly defined by the downstream part (i.e. that in UsBX in proper "sense" orientation). These two putative promoters will be analyzed further by standard procedures.

### Deletion analysis of the 5' promoter region and first intron promoter region of the p75 TNF-R gene

Deletional analysis of the promoter regions containing the 5' upstream promoter and the intron promoter were carried out to ascertain more precisely the sequence regions defining the active promoters. For this purpose, additional constructs were prepared, as detailed above, in which various deletion fragments of the 5' upstream promoter region and various deletion fragments of the 3' end of the first intron promoter region were inserted into the expression vectors carrying the luciferase reporter gene, and tested for promoter activity in the transfected BHK cells. The deletion fragments were generated by utilizing the above mentioned vectors containing the full-length 5' upstream promoter region and intron promoter region and then deleting from the 5' ends of these promoter regions, various portions to yield deletion fragments thereof, all by standard procedures. The functional assay of these deletion fragments for promoter activity was carried out as detailed above.

The deletion fragments of the 5' upstream promoter region included: (i) a fragment designated "GN2BX" extending from nucleotide no. 197 to nucleotide no. 2086 of the sequence of Fig. 2A; (ii) a fragment designated "75" extending from nucleotide no. 682 to nucleotide no. 2086 of the sequence of Fig. 2A; (iii) a fragment designated "PIII" extending from nucleotide no. 709 to nucleotide no. 2086 of the sequence of Fig. 2A; (iv) a fragment designated "76" extending from nucleotide no. 1335 to nucleotide no. 2086 of the sequence of Fig. 2A; and (v) a fragment designated "PI" extending from nucleotide no. 1527 to nucleotide no. 2086 of the sequence of Fig. 2A.

The deletion fragments of the intron promoter region included: (vi) a fragment designated "74" extending from nucleotide no. 872 to nucleotide no. 1827 of the sequence of Fig. 2B; and (vii) a fragment designated "UIIP" extending from nucleotide no. 1569 to nucleotide no. 1827 of the sequence of Fig. 2B.

All of the above deletion fragment (i)-(vii) were cloned into the expression vectors by standard methods and in a way that all were in the "sense" orientation.

From the results (not shown) it was apparent that:
(a) For the 5' upstream promoter region, the deletion fragments (i)-(iv), i.e. "GN2BX", "75", "PIII" and "76" all had full promoter activity, comparable to that of the non-deleted, cloned promoter region (nucleotides 1-2089 of Fig. 2A, see above), but however, the deletion fragment (v), i.e. "PI" had a very reduced promoter activity.
(b) For the intron region promoter, the deletion fragment (vi), i.e. "74" showed very little promoter activity, this being comparable to the very low (if any) promoter activity of the cloned full-length fragment ("USBX", nucleotides 1-1827 of Fig. 2B) and of the other cloned fragment having only the 5' region of the intron promoter region (nucleotides 1-874 of Fig. 2B). However, the deletion fragment (vii), i.e. "UIIP" had a very significant increased promoter activity (comparable to those of "75", "PIII" and "76" noted above).

From the above results it may therefore be concluded that:
(a) For the 5' upstream promoter, the 5' end of the promoter is located between the 5' end of deletion fragment (iv) i.e. "76" (nucleotide no. 1335 of Fig. 2A) and the 5' end of deletion fragment no. (v) i.e. "PI" (nucleotide no. 1527 of Fig. 2A).
(b) For the first intron promoter region, there is only one promoter in the approx. 1.9 kb fragment, at the 3' end of the intron. The 5' end of this promoter is located between the 5' end of deletion fragment (vii) i.e. "UIIP" (nucleotide no. 1569 of Fig. 2B) and 1768 (start of new leader, see above). Moreover, the results also provided the surprising observation that there is an inhibitory region which is located between the 5' end of the full-length intron fragment (nucleotide 1 of Fig. 2B) and the 5' end of "UIIP" (nucleotide 1569 of Fig. 2B) . At least part of this inhibitory region sequence is located between the 5' end of deletion fragment (vii) i.e. "74" (nucleotide 872 of Fig. 2B) and the 5' end of "UIIP" (nucleotide 1569 of Fig. 2B).

The 5' upstream promoter has thus now been more fully defined to be present within the region defined by nucleotides 1335 and 1527 of Fig. 2A.

For the intron promoter the results indicate that in the region between nucleotide 1 of Fig. 2B and the 5' end of "74" (nucleotide 872 of Fig. 2B) there is no promoter bur rather there exists a transcription inhibitory region. Further, this inhibitory region appears to extend into the "74" fragment up to the 5' end (nucleotide 1569 of Fig. 2B) of the smaller "UIIP" fragment.

Thus, the intron promoter region is from 5' end of "UIIP" (nucleotide 1569 of Fig. 2B to nucleotide 1768 of Fig. 2B), the start of the new leader.

This new intronic promoter is also unique in that besides being present in an intron it is also apparently devoid of a larger number of motifs usually present in promoters, all the observed motifs except for one TCC repeat (see above and Fig. 2B) being upstream in the region that is now believed to be inhibitory.

The existence of this promoter in the intron makes it likely that an alternative form of the p75-TNF-R is expressed, namely one having the presumed new leader sequence followed by the regions encoded by exons 2 and the remaining exons of the p75-TNF-R gene. This alternative form of p75 TNF-R is thus devoid of the region encoded by the first exon, namely the N-terminal part of the extracellular domain region which is known to be directly upstream of the cysteine-rich region (the TNF-binding region).

It is known in other proteins (e.g. dystrophin) that when the protein is transcribed from different promoters there occurs concurrently a mechanism of alternative splicing. Accordingly, it is possible that the putative new form of p75-TNF-R transcribed from the intronic promoter also undergoes alternative splicing to yield a final product which is a soluble form of p75-TNF-R that contains only the cysteine-rich domain; which is the TNF-binding region. This mechanism of alternative splicing of the p75 TNF-R transcript transcribed from the intronic promoter may be another way (besides the previously described protease cleavage process) in which the naturally-occurring soluble form of p75-TNF-R is produced.

The finding of the transcription inhibitory region in the intron upstream of the intronic promoter indicates the possibility that this inhibitory region serves as a modulator of the expression of the p75 TNF-R gene, and more particularly, of the putative new form of the p75-TNF-R. This modulation may be via the action of cytokines, in particular, TNF and IL-1, or differentiation-induced changes. Preliminary results (not shown) have indicated that, in fact, TNF is capable of enhancing the function of the intronic promoter by suppressing the inhibitory region. Further, there also appears to be cell-specific expression of the intronic promoter, some cells having naturally high expression of this promoter and others not, these differences being related to differentiation changes which have apparently led to the inactivation of the inhibitory region in some cell types.

Accordingly, the present invention also concerns the above inhibitory region.

### EXAMPLE 4

### Purification of transcription factors binding to the promoter region

Functional motifs in the promoter region (i.e. 5' upstream and first intron promoters) and the inhibitory region present in the first intron region upstream of the intron promoter (see Example 3) can be identified by step-wise deletion of nucleic acid sequence from the 3' and/or 5' end of the promoter and/or inhibitory regions by conventional means (Erase-a-Base kit, Promega Corp.). The deleted promoter or inhibitory region fragments are then tested for activity. Likewise, internal sequences can be deleted or changed by *in vitro* mutagenesis or linker scanning (31). Motifs that bind activating transcription factors are revealed by a loss of promoter activity when deleted or mutated. Conversely, motifs that bind transcription factors which suppress promoter activity are identified by mutated or deleted promoter fragments which have increased activity, compared to the wild-type promoter. Likewise, motifs concerned with activation or inhibition of the transcription inhibitory region can also be identified, which bind inhibitory factors or inhibition suppressor factors. A detailed analysis of these motifs is then carried out by chemical synthesis of oligonucleotides with the sequence of the original motif, and mutated forms of it. These oligonucleotides are linked to the promoter fragments lacking the corresponding motifs, and the resulting construct is tested for promoter activity. If the original activity is restored, the motif can be regarded as functionally unchanged, i.e. those mutations that have been introduced into the motif, do not interfere with its function. On the other hand, if less promoter activity is observed with a mutated motif, it can be concluded that the nucleotides which were changed compared to the wild-type motif, are essential for its function.

Thus, all of the various motifs set forth in Example 2 above, within the 5' upstream and intron promoter regions, can be subjected to the above analysis procedures to determine which are the fully functional ones.

Once a transcription factor binding motif has been identified, the corresponding transcription factor is isolated. For this purpose, extracts from several sources are screened for high expression of that transcription factor. The amount of transcription factor present can be measured by gel shift assays, using the above described oligonucleotides containing the sequence of the functional motif at 5'-end-labeled, ds-DNA probes.

Having identified an abundant source of the transcription factor, the conditions that are required for optimum binding can be defined. Different chemical parameters, such as pH, presence of various mono- and divalent cations, salt concentration and the presence of reducing agents, e.g. DTT or mercaptoethanol are adjusted to achieve this goal.

Having established optimal binding conditions for the transcription factor, purification is carried out by conventional means, e.g. by salt precipitation, phosphocellulose and/or DEAE chromatography. An enriched precipitation of the transcription factor is then purified further on a DNA affinity column, in which the oligonucleotide containing the corresponding motif is bound to an insoluble matrix, and the transcription factor-containing solution is passed over the column under conditions optimal for binding. After washing off contaminants, the purified transcription factor is eluted by conditions which do not allow DNA binding, e.g. pH shift, changed salt concentration, or chelation of divalent salts necessary for DNA binding (usually Zn⁺⁺).

Having purified the transcription factor allows the application of "reverse genetics" on that molecule:
protein sequencing, cDNA cloning using degenerated oligonucleotides corresponding to protein sequence and
finally, cloning of the gene encoding the transcription factor by screening genomic libraries using the cDNA as a probe.

Having all these tools: genomic clones, cDNA and purified transcription factors, allows to define ways to regulate the activity of the transcription factor by one of the following means: (1) influencing its promoter; (2) influencing its binding to the target in the p75 gene promoter; or (3) modulating its activity.

A detailed procedure for (1) is given in Example 5. Methods (2) and (3) can be achieved by screening a large number of drugs for interference with the function of this transcription factor.

In a similar fashion, an analogous procedure as detailed above may be carried out to identify, clone, characterize and regulate the activity of the factors which recognize the inhibitory region present in the first intron upstream of the intron promoter (see Example 3).

### EXAMPLE 5

### Modulation of promoter activity by specific sequence regions

The activity of a promoter can be regulated by scavenging transcription factors which are in short supply. This can be done by expressing multiple copies of the corresponding motifs to which the transcription factors bind. This mechanism has recently been demonstrated by Pai et al (34), who expressed and amplified the negative promoter domain of the c-myc promoter in the hamster CHO cell line. Following that, the authors observed increased expression of hamster c-myc and the corresponding changes in cell growth and morphology induced by myc protein. Much in the same way, it is possible to amplify promoter regions which activate and enhance promoter activity, and by that decrease the expression of the corresponding protein.

For the p75 promoter, either the whole extreme 5' region promoter or the whole promoter in the first intron (see Example 2), or parts thereof which have been identified as negative or positive regulatory domains, can be excised from the promoter sequence by restriction digest or exonuclease deletion of irrelevant sequences. The fragments obtained are then linked to a vector that allows gene amplification, and transfected into a cell line, e.g. CHO cells, which allows selection for amplified vector sequences. After selection and amplification, the obtained clones of CHO cells are checked for p75 gene expression on the mRNA and protein level. In addition, the function of the receptors in checked by cytotoxicity assay with TNF or with TNF mimicking antibodies as described in Israel Patent Application No. 103051.

Having established promoter regions which, upon amplification in this system, modulate the activity of the p75 receptor, these same regions are introduced into cells which do not allow selection for amplified gene products in two ways:
(1) Coexpression of promoter regions linked to a vector which contains a viral origin or replication (e.g. SV40 or EBNA), with a vector which expresses T antigen (of SV40), or EBNA antigen. This allows the replication of high numbers of episomal copies of the introduced promoter fragment in the nucleus of the target cell and thus mimics the effect of DNA amplification of integrated sequences.
(2) Chemical synthesis of a ds oligonucleotide comprising the promoter domain and application of sufficient amounts of that oligonucleotide to cells makes it likewise possible to scavenge the corresponding transcription factors and thus influence promoter activity. The chemistry of the oligonucleotides has to be changed in order to (a) make the oligonucleotide more lipophilic, so that it can pass the cytoplasmic membrane, and (b) enhance its stability in order to minimize degradation. This is done by conventional means. e.g. by using phosphothioate-coupled oligonucleotides.

### REFERENCES

1. Tracey, J.T. et al. (1987) Nature 330:662-664.
2. Piquet, P.F. et al. (1987) J. Exp. Med. 166:1280-1289.
3. Beutler, B. and Cerami, C. (1987) NEJM, 316:379-385.
4. Hohmann, H.-P. et al. (1989) J. Biol. Chem. 264:14927-14934.
5. Engelmann, H. et al. (1990) J. Biol. Chem. 265:1531-1536.
6. Brockhause, M. et al. (1990) Proc. Natl. Acad. Sci. USA 87:3127-3131.
7. Loetscher, H. et al. (1990) Cell 61:351-359.
8. Schall, T.J. et al. (1990) Cell 61:361-370.
9. Nophar, Y. et al. (1990) EMBO J. 9:3269-3278.
10. Smith, C.A. et al. (1990) Science 248:1019-1023.
11. Heller, R.A. et al. (1990) Proc. Natl. Acad. Sci. USA 87:6151-6155.
12. Aggarwal, B.B. et al. (1985) Nature 318:665-667.
13. Israel, S. et al. (1986) Immunol. Lett. 12:217-224.
14. Tsujimoto, M. et al. (1986) Biochem. Biophys. Res. Commun. 137:1094-1100.
15. Ruggiero, V.J. et al. (1986) J. Immunol. 136:2445.
16. Holtman, H. and D. Wallach (1987) J. Immunol. 139:1161-1167.
17. Ding, A.H. et al. (1989) J. Biol. Chem. 264:3924.
18. Porteu, F. and Nathan, C. (1990) J. Exp. Med. 17:599-607.
19. Porteu, F. et al. (1991) J. Biol. Chem. 266:18846.
20. Ware, C.F. et al. (1991) J. Immunol. 147:4229.
21. Erikstein, B.K. et al. (1991) Eur. J. Immunol. 21:1033.
22. Winzen, R. et al. (1992) J. Immunol. 148:3454.
23. Espevik, T. et al. (1990) J. Exp. Med. 171:415-426.
24. Engelmann, H. et al. (1990) J. Biol. Chem. 265:14497-14504.
25. Thoma, B. et al. (1990) J. Exp. Med. 172:1019-1023.
26. Tartaglia, L.A. et al. (1991) Proc. Natl. Acad. Sci. USA 88:9292-9296.
27. Gehr, G. et al. (1992) J. Immunol. 149:911.
28. Heller, R.A. et al. (1992) Cell 70:47.
29. Brakebusch, C. et al. (1992) EMBO J. 11:943-950.
30. Vandenabeele, P. et al. (1992) J. Exp. Med. 176:1015.
31. Sambrook, J. et al. (1989) Molecular Cloning : A Laboratory Manual, Cold Spring Harbor Laboratory Press, Cold Spring Harbor, N.Y.
32. Lenardo, M.J. and Baltimore, D. (1989) Cell 58:227-229.
33. Imagawa, M. et al. (1987) Cell 51:251-260.
34. Pai et al. (1992) J. Biol. Chem. 267:12428-12431.
35. Kohno T. et al, (1990) Proc. Natl. Acad. Sci. USA, 87:8331-8335
36. Seto E. et al (1991) Nature 354:241-245
37. Kageyama R. et al. (1988) Proc. Natl. Acad. Sci. USA 85:217-224.
38. Kageyama R. et al. (1989) J. Biol. Chem. 264:15508-14.
39. Fashena S.J. et al. (1992) Mol. Cell Biol. 12:894-903.
40. Murre C. et al. (1989) Cell 56:777-83.
41. Shannon M.T. et al. (1990) Mol Cell Biol. 10:2950-2959.
42. Anisowicz A. et al. (1991) J. Immunol. 147:520-527.
43. Winzen R. et al. (1993) J. Immunol. 150:4346-53.
44. Kalthoff H. et al. (1993) J. Biol. Chem. 268:2762-6.
45. Fujita T. et al. (1987) Cell 49:357-67.
46. Harroch S. et al. (1993) J. Biol. Chem. 268:9092-9097.
47. Mitchell P. et al. (1987) Cell 50:847-61.
48. Scheurich P. et al. (1989) J. Exp. Med. 170:947-58.
49. Hohmann H-P. et al. (1990) - J. Biol. Chem. 265:22409-17.
50. Aggarwal B. B. et al. (1993) Lymph. Cyt. Res. 12:149-58.
51. Lewis E. et al. (1987) Proc. Natl. Acad. Sci. USA 84:3550-4.
52. Ullman K.S. et al. (1991) Science 254:558-62.
53. Corcoran L.M. et al. (1993) Genes Dev. 7:570-82.
54. Abe E. et al. (1992) Proc. Natl. Acad. Sci. USA 89:2864-8.
55. Aebi M. and Weismann C. (1987) TIG 3:102-107.
56. Kemper O. and Wallach, D. (1993) Gene 134:209-216.
57. Shannon M. F. et al. (1988) Proc. Natl. Acad. Sci. USA 85:674-678.
58. Okret S. et al. (1986) Proc. Natl. Acad. Sci. USA 83:5899-903.
59. Chambaut-Geurin, A-M. and Thomopoulos, P. (1991) Eur. Cyt. Netw. 2:355-60
60. GCG Computer Program, Genetics Computer Group, 575 Science Drive, Madison, Wisconsin USA 53711 (1991).
61. Kozak M. (1987) Nucleic Acids Res. 15:8125-8148.
62. Ausubel F.M. et al. (1989) Current Protocols in Molecular Biology, John Wiley, N.Y. 15.4.1
63. Nordeen S.K. (1988) Biotechniques 6:454-8.
64. Boshart K. et al. (1992) Gene 110:129-130.
65. Koizumi, M. et al. (1993) Biol. Pharm. Bull. (Japan) 16:879-83.
66. Crisell, P. et al. (1993) Nucleic Acids Res. 21:5251-5.
67. Barinaga, M. (1993) Science 262:1512-4.
68. Cantor, G.H. et al. (1993) Proc. Natl. Acad. Sci. USA 90:10932-6.
69. Shimayama, T. et al. (1993) Nucleic Acids Symp. Ser. 29:177-8.
70. Joseph, S. and Burke, J.M. (1993) J. Biol. Chem. 268:24515-8.
71. Chen, C.J. et al. (1992) Ann N.Y. Acad. Sci. 660:271-3.
72. Shore, S.K. et al. (1993) Oncogene 8:3183-8.
73. Zhao, J.J. and Pick, L. (1993) Nature 365:448-51.
74. Kemper, O. et al. (1993) Gene 134: 209 - 216.

## Claims

1. A promoter sequence of the human p75 tumor necrosis factor receptor (TNF-R) gene which is a 5' upstream promoter sequence, comprising at least the sequence extending between nucleotide 1335 and 1527 of the nucleotide sequence set forth in Figure 2A.

2. A promoter sequence of the human p75 TNF-R gene which is located in the first intron between the first and second exons comprising at least the sequence extending between nucleotides 1569 and 1768 of the sequence set forth in Figure 2B.

3. A sequence containing a transcription inhibitory region that is located within the sequence of the 1.9 kbp Smal fragment of the genomic clone encoding the human p75 TNF-R genes as shown in Figure 1 upstream of the intron promoter sequence, wherein said transcription inhibitory region comprises the sequence extending between nucleotides 1 and 1569 of the sequence set forth in Figure 2B.

## Patentansprüche

1. Promotersequenz des menschlichen p75-Tumornekrosefaktor-Rezeptor (TNF-R)-Gens, die eine 5'stromaufwärts gelegene Promotersequenz ist und wenigstens die Sequenz zwischen Nucleotid 1335 und 1527 der Nucleotidsequenz aus Figur 2A umfasst.

2. Promotersequenz des menschlichen p75-TNF-R-Gens, die sich in dem ersten Intron zwischen dem ersten und dem zweiten Exon befindet und wenigstens die Sequenz zwischen den Nucleotiden 1569 und 1768 der Sequenz aus Figur 2B umfasst.

3. Sequenz, enthaltend eine Transkriptions-inhibierende Region, die sich in der Sequenz des 1,9 kbp Smal-Fragments des genomischen Clons, der die menschlichen p75-TNF-R-Gene dargestellt in Figur 1 codiert, stromaufwärts der Intron-Promotersequenz befindet, wobei die Transkriptions-inhibierende Region die Sequenz zwischen Nucleotid 1 und 1569 der Sequenz aus Figur 2B umfasst.

## Revendications

1. Séquence promoteur du gène de récepteur de facteur de nécrose tumorale (TNF-R) p75 humain, qui est une séquence promoteur amont 5', comprenant au moins la séquence qui s'étend entre les nucléotides n° 1335 et 1527 de la séquence de nucléotides présentée sur la figure 2A.

2. Séquence promoteur du gène de TNF-R p75 humain, qui est située dans le premier intron, entre le premier exon et le deuxième exon, compre-nant au moins la séquence qui s'étend entre les nucléotides n° 1569 et 1768 de la séquence présentée sur la figure 2B.

3. Séquence contenant un domaine d'inhibition de la transcription, qui est située dans la séquence du fragment SmaI de 1,9 kbp du clone géno-mique codant les gènes de TNF-R p75 humain, représenté sur la figure 1, en amont de la séquence promoteur de l'intron, lequel domaine d'inhibition de la transcription comprend la séquence qui s'étend entre les nucléotides n° 1 et 1569 de la séquence présentée sur la figure 2B.
